# FASCICULE DE BREVET EUROPEEN

(11) **EP 2 897 562 B1**
(45) Date de publication et mention de la délivrance du brevet: **09.11.2016**
(21) Numéro de dépôt: 13774722.6
(22) Date de dépôt: 04.09.2013
(51) Int. Cl.: D04B 15/18, A61F 13/08, D04B 1/26

(54) **PROCEDE DE FABRICATION D'UN ARTICLE TUBULAIRE DE COMPRESSION ET ARTICLE AINSI OBTENU**
VERFAHREN ZUR HERSTELLUNG EINES RÖHRENFÖRMIGEN KOMPRESSIONSARTIKELS UND HERGESTELLTER ARTIKEL
METHOD FOR PRODUCING A TUBULAR COMPRESSION ITEM, AND ITEM THEREBY OBTAINED

(30) Priorité: 19.09.2012 FR 1258771
(43) Date de publication de la demande: 29.07.2015
(73) Titulaire: Thuasne, 92300 Levallois Perret (FR)
(72) Inventeur: CONVERT, Reynald, F-42800 Saint Martin La Plaine (FR); RUER, Aurélia, F-42240 Saint Maurice En Gourgois (FR); CATTIAUX, Gérard, F-42480 La Fouillouse (FR); MOTET, Pascal, F-42100 Saint Etienne (FR)
(74) Mandataire: Balesta, Pierre
(86) Numéro de dépôt international: PCT/FR2013/052039
(87) Numéro de publication internationale: WO 2014/044945

(56) Documents cités:
- EP-A1- 2 436 276
- WO-A1-2012/101618
- FR-A1- 2 852 509

## Description

### Arrière-plan de l'invention

La présente invention concerne le domaine technique des procédés de fabrication des articles tubulaires de compression, en particulier des articles exerçant une compression dégressive depuis la cheville vers le haut de la jambe.

Les articles de compression, chaussette ou mi-bas, bas, collant ou encore manchon, sont utilisés pour prévenir ou soigner les problèmes de circulation veineuse notamment au niveau du membre inférieur ou supérieur, ainsi que les pathologies liées aux dysfonctionnements du système lymphatique et réduire les oedèmes. Les troubles veineux peuvent avoir plusieurs origines, parmi celles-ci on trouve notamment : une rigidification de la paroi veineuse, une altération des valvules ou encore une augmentation du diamètre des veines.

La pression locale exercée sur un membre par un article à effet compressif est fonction notamment des caractéristiques de force-allongement dudit article.

La pression exercée sur un membre se calcule par la loi de Laplace suivante : P [Pa ou mmHg] = (T [N] X n / (L [m] X R [m]).

P représente la pression exercée en un point donné du membre considéré.

L est la largeur de la zone considérée du membre et n représente le nombre de couches de l'article à effet compressif disposées sur ladite zone.

T est la tension, exprimée en Newtons, exercée par ledit article lorsqu'il est enfilé sur le membre inférieur ou supérieur.

R est le rayon de courbure au point considéré du membre inférieur ou supérieur.

Plus la déficience du système veineux est importante, plus le sang présente des difficultés à refluer depuis la cheville vers le coeur, et plus la pression à exercer au niveau de la cheville est importante.

A titre d'exemple dans le système français, les niveaux de compression peuvent être répartis ainsi :

| | |
|---|---|
| Classe I : 13 à 20 hPa | Classe II : 20,1 à 27 hPa |
| Classe III : 27,1 hPa à 48 hPa | Classe IV : plus de 48 hPa |

Les articles de compression, notamment de classe élevée, sont difficiles à enfiler par le patient, notamment lorsque celui-ci souffre d'une mobilité réduite.

Habituellement, les bas de compression (ou à effet compressif) médicaux sont constitués de deux fils, à savoir un fil de trame et un fil dit de maille. Le fil de trame est un fil élastique dont le parcours est quasiment linéaire dans la direction des rangées de mailles des articles de compression. Le fil de trame permet d'attribuer l'effet compressif à l'article de compression. Le fil de maille, également désigné fil de fond, donne à l'article de compression tricoté ses dimensions ainsi que ses propriétés de confort et esthétiques.

Dans l'état de la technique, les articles de compression médicaux sont tricotés sur des métiers circulaires simple cylindre avec un plateau de report ou sur des métiers rectilignes double fontures, la productivité étant meilleure avec les métiers circulaires.

Dans le cas de la fabrication d'un article de compression médical sur un métier à tricoter simple cylindre avec plateau de report, la maille employée est généralement du type jersey endroit. Afin que le fil de trame soit solidaire de l'article de compression tricoté, il réalise des charges, voire parfois des mailles, sur le fil de maille, le fil de trame est alors bloqué dans les pieds de tout ou partie des mailles tricotées avec le fil de maille.

Lorsque l'on souhaite attribuer un effet de « côte » à l'article de compression, le fil de maille ou fil de fond réalise des rangées de mailles de jersey endroit tandis que le fil de trame réalise des charges avec des rapports différents. Plus la côte est large, plus le flotté réalisé par le fil de trame sur l'envers du tricot l'est également. Ces flottés de fil de trame étant apparents sur la face envers de l'article tricoté forment des zones d'accroche et de frottements pour la peau lors de l'enfilage de l'article sur la jambe compliquant ainsi son positionnement correct.

Les zones de charge du fil de trame constituent des zones de blocage du fil de trame dans la direction transversale de l'article tubulaire tricoté limitant son élasticité, et rendent encore plus difficile la mise en place et le retrait de l'article de compression.

Il existe ainsi un besoin pour un article de compression n'ayant pas de zones d'accroche et/ou de frottement avec la peau et très facile d'enfilage tout en améliorant le confort dudit article.

En outre, la finition de la bordure ou zone périphérique supérieure en aval de la tige de l'article de compression à partir de laquelle le pied est inséré dans l'article, implique de bloquer les fils de maille et de trame afin d'éviter tout effilochage.

Une première technique de finition consiste tout simplement à terminer ladite zone périphérique supérieure par couture. Cette technique a pour inconvénient que le revers est peu esthétique, et qu'elle limite l'élasticité de ladite zone comparativement au reste de l'article de compression ce qui génère une bordure qui ne reste pas en position sur la jambe, a tendance à glisser et rouler, et peut s'avérer inconfortable.

Une seconde technique consiste à réaliser un revers par tricotage au niveau de ladite zone périphérique supérieure, ledit revers a une armure de tricotage proche de celle du reste de l'article de compression mais il est obtenu par report de mailles entre le plateau et le cylindre, plus précisément entre les platines de report et les aiguilles, formant ainsi une double épaisseur. Le revers ainsi formé exerce une pression sur la jambe plus importante que celle exercée par la tige. Le revers peut même, dans certains cas, exercer un effet garrot. Ce revers formé d'une double épaisseur est également plus chaud que le restant dudit article de compression. Enfin, selon la morphologie du patient, il a tendance à glisser.

Il existe donc un besoin pour un article tubulaire de compression exerçant une force de compression de la cheville jusqu'au mollet parfaitement dégressive et dont la bordure ou zone périphérique supérieure ne glisse ou ne roule pas sur la jambe et ne soit pas plus épaisse, i.e. ayant un masse surfacique (g/m²) qui ne soit pas plus importante que le restant de l'article de compression.

### Objet et résumé de l'invention

La présente invention pallie tout ou partie des problèmes précités en ce qu'elle a pour objet selon un premier aspect, un procédé de fabrication d'un article tubulaire de compression, du type chaussette , bas ou mi-bas, ou collant, ayant au moins une tige, une pointe de pied, un talon, un pied et un bord côte dans le prolongement de la tige délimitant une ouverture pour l'introduction du pied dans ledit article, comprenant les étapes suivantes :
a. Une première étape de tricotage de la tige, du pied et du bord côte avec au moins un fil de maille sur un métier à tricoter double cylindres comprenant un cylindre supérieur et un cylindre inférieur travaillant chacun avec (m) aiguilles, au cours de laquelle plusieurs rangées de mailles en côte (n)*(p), (n')*(p') et (n")*(p") sont tricotées respectivement pour la tige, le pied et le bord côte (6), (m), (n), (n'), (n"), (p), (p') et (p") étant des nombres entiers supérieurs ou égaux à 1,
b. Une étape d'insertion au cours de la première étape de tricotage d'un fil de trame élastique entre deux rangées de mailles côtelées (n)*(p) et (n')*(p') toutes les 1/1 à 1/5 rangées de mailles de la tige et du pied et toutes les 1/2 à 1/5 rangées de mailles du bord côte, sur au moins 50 % en nombre du nombre (m) d'aiguilles sans former de charge, ni de maille, la densité en fil de trame dans le bord côte étant inférieure ou égale à la densité en fil de trame dans la tige,
c. Une seconde étape de tricotage de la pointe de pied et du talon sur ledit métier à tricoter double cylindres avec au moins un fil de maille et éventuellement un fil de trame élastique.

Avantageusement, un métier à tricoter double cylindres permet de poser le fil de trame entre deux rangées de mailles consécutives sans le bloquer en le passant à travers les pieds des mailles de sorte que le fil de trame se retrouve entre les mailles tricotées sur l'endroit formant la face extérieure de l'article et les mailles tricotées sur l'envers formant la face intérieure de l'article de compression. Dans les portions de tige et de pied tricotées avec un fil de maille selon des mailles côtelées et un fil de trame élastique, le fil de trame forme ainsi en quelque sorte une couche intermédiaire disposée entre les faces extérieure et intérieure et ne vient pas en contact directement avec la peau évitant ainsi de former des zones d'accroche et/ou de frottements avec la peau. L'enfilage de l'article est ainsi facilité et le confort au contact dudit article est amélioré.

En outre, lorsque le fil de trame est simplement posé entre deux rangées de mailles consécutives, il n'est pas bloqué dans les pieds des mailles envers et/ou endroits de sorte que l'élasticité des zones ainsi tricotées est plus importante pour un même effet compressif. Cette disposition améliore encore le confort, facilite l'enfilage et limite le glissement sur la jambe dudit article.

Enfin, les côtes selon l'invention sont des « vraies » mailles côtelées. En effet, dans l'état de la technique lorsqu'on utilise un métier à tricoter simple cylindre, les côtes sont formées par rapprochement des zones de charge du fil de trame, et donc en quelque sorte par froncement des zones tricotées disposées entre ces zones de charge.

On emploie dans le présent texte les termes « mailles côtelées » ou « mailles côte » indifféremment.

La densité de fil de trame correspond à l'insertion d'un fil de trame toutes les a/b rangées de mailles, a et b étant des nombres entiers supérieurs ou égaux à 1.

Dans le cadre de la présente invention, la tige comprend plusieurs rangées de mailles côte (n)*(p) tandis que le pied et le bord côte comprennent des rangées de mailles côtelées respectivement (n')*(p') et (n")*(p"), avec n, n', n", p, p', et p" étant des nombres entiers supérieures ou égaux à 1. En effet, la structure côtelée peut être différente entre la tige, le pied et le bord côte. De même, la structure côtelée peut être différente à l'intérieur même de la tige, du pied et du bord côte. Ainsi, à titre d'exemple, la tige peut présenter des mailles côtelées 2/4 alternées avec des mailles côtelées 1/1.

Le fil de maille forme des mailles côtelées, mais peut également former des mailles jersey endroit et/ou envers selon l'effet recherché.

Le métier à tricoter utilisé dans le cadre de la présente invention est un métier à tricoter double cylindres, i.e. ayant un cylindre supérieur et un cylindre inférieur travaillant chacun avec le même nombre d'aiguilles (m).

Le fil de trame est de préférence disposé entre deux rangées de mailles consécutives sur au moins 50 % en nombre du nombre des aiguilles travaillées (m), il peut ainsi être chargé ou maillé selon l'effet recherché sur au plus 50 % en nombre des aiguilles restantes.

De préférence, le fil de trame est inséré entre deux rangées de mailles consécutives sur au moins 75 % en nombre du nombre (m) d'aiguilles travaillées sur les cylindres supérieure et inférieure, encore de préférence sur au moins 90 % en nombre du nombre (m) des aiguilles travaillées, et encore de préférence sur toutes les aiguilles travaillées sur les cylindres supérieure et inférieure sans former de charge, ni de maille.

Avantageusement, le métier à tricoter double cylindres permet de terminer l'ouverture d'introduction sans avoir recours à un revers comme cela est le cas avec les métiers simple cylindre. Le bord côte ne présente pas de repli. En outre, la densité en fil de trame dans le bord côte est inférieure ou égale à la densité en fil de trame dans la tige ce qui permet de maintenir une parfaite dégressivité de la compression exercée et évite au bord côte d'exercer un effet garrot.

Ledit bord côte peut comprendre des rangées de mailles endroit et/ou envers, formées à partir du fil de maille et éventuellement du fil de trame inséré en trame dans les pieds de mailles.

De préférence, le bord côte ne comprend que des mailles côtelées (n")*(p"), ce qui permet de supprimer totalement la tendance à roulotter de ladite ouverture et d'éviter le glissement de ce dernier sur la peau contrairement à un revers formé de mailles jersey.

De préférence, le fil de trame élastique est inséré entre deux rangées de mailles côte toutes les 1/2 à 1/5 rangées de mailles sur au moins 75 % en nombre, de préférence sur au moins 90 % en nombre, du nombre (m) d'aiguilles sans former de charge ni de maille.

Dans une sous-variante, le fil de trame élastique est inséré entre deux rangées de mailles côte toutes les 1/2 à 1/5 rangées de mailles sur toutes les aiguilles travaillées (m) sans former de charge, ni de maille.

L'article tubulaire de compression selon l'invention présente une direction longitudinale (L) et une direction transversale (T) correspondant respectivement aux colonnes de mailles et aux rangées de mailles.

Selon la force de compression recherchée, le fil de trame peut être inséré entre toutes les rangées de mailles ou toutes les 1/2 à 1/5 rangées de mailles.

On comprend par fil élastique, un fil ayant un allongement à rupture supérieure ou égale à 100 %, de préférence supérieure ou égale à 200 %, et encore de préférence supérieure ou égale à 300 %. Ces valeurs peuvent être déterminées par exemple à l'aide de la norme NF EN ISO 2062 de janvier 2010.

Les titrages indiqués dans le présent texte peuvent être mesurés à l'aide de la norme NF EN ISO 2060 de juin 1995.

On comprend par maille chargée, la disposition d'un fil dans une boucle sans que ce fil ne forme lui-même une boucle.

Le fil de maille selon l'invention peut être élastique.

Dans une variante, la seconde étape de tricotage ne comprend pas l'insertion en trame d'un fil de trame élastique.

Il n'est pas nécessaire d'exercer un effet de compression particulier dans la pointe de pied ou le talon de sorte que seul le fil de maille est tricoté dans ces zones de l'article.

Dans une variante, le procédé selon l'invention comprend au cours de ladite première étape et/ou de ladite seconde étape, une sous-étape de tricotage du fil de maille pour la formation d'une ou plusieurs rangées de mailles en jersey avant et/ou arrière.

Dans une variante, le bord côte a une hauteur (h) au minimum de 1 mm.

De préférence, le bord côte a une hauteur (h) au minimum de 20 mm, et encore de préférence au minimum de 50 mm.

Dans une variante, la tige présente deux zones circulaires distinctes A et B consécutives. En outre, au cours des première et seconde étapes de tricotage, la tension exercée sur le fil de trame élastique lors du tricotage de la zone A est supérieure à la tension exercée sur le fil de trame élastique dans la zone B en sorte que la circonférence de la zone circulaire A soit inférieure à la circonférence de la zone circulaire B.

Cette disposition permet avantageusement de former une tige ayant une forme générale tronconique dont la dégressivité de la compression exercée est parfaitement homogène.

La tension exercée (daN) est celle appliquée sur le fil de trame avant son insertion dans le cylindre supérieur ou inférieur. Cette tension peut être mesurée à l'aide d'un tensiomètre mécanique.

Dans une variante, le procédé selon l'invention comprend au cours de la première étape, et/ou de la seconde étape, une sous-étape au-cours de laquelle le fil de trame élastique effectue une ou plusieurs charges.

Dans une variante, le fil de trame élastique est inséré en trame au cours de la première étape toutes les 1/1 à 1/2 rangées de mailles.

L'invention a également pour objet, selon un deuxième aspect, un article tubulaire à effet compressif, du type chaussette, bas ou mi-bas, ou collant, ayant au moins une tige, une pointe de pied, un talon et un pied, et un bord côte dans le prolongement de la tige délimitant une ouverture pour l'introduction du pied dans ledit article , obtenu par la mise en oeuvre du procédé selon l'une quelconque des variantes de réalisation précitées, comprenant un fil de maille tricoté en côte (n)*(p), (n')*(p') et (n") *(p") respectivement dans la tige, le pied et le bord côte, (n), (n'), (n"), (p), (p') et (p") étant des nombres entiers supérieurs ou égaux à 1, et un fil de trame élastique disposé entre deux rangées de mailles consécutives toutes les 1/1 à 1/5 rangées de mailles dans la tige, le pied et le bord côte, sur au moins 50 %, de préférence sur au moins 75 %, encore de préférence sur au moins 90 %, en nombre du nombre de colonnes de mailles sans former de charge, ni de maille, la densité en fil de trame dans le bord côte étant inférieure ou égale à la densité en fil de trame dans la tige,

Dans une sous-variante, le fil de trame élastique est disposé entre deux rangées de mailles consécutives toutes les 1/1 à 1/5 rangées de mailles dans la tige, le pied et éventuellement le bord côte sur toutes les colonnes de maille sans former de charge ni de maille.

Dans une variante, le fil de trame élastique est inséré en trame dans la tige et le pied toutes les 1/1 à 1/2 rangées de mailles.

Dans une variante, le fil de trame est constitué d'un fil d'âme élastique recouvert d'un ou de plusieurs fils de couverture, notamment par guipage, lesdits fils de couverture étant de préférence non élastiques.

De préférence, le fil d'âme élastique est un fil d'élasthanne ou d'élastodiène.

Dans une variante, le fil de trame élastique a un titrage compris entre 150 dtex et 2000 dtex, de préférence entre 300 dtex et 1500 dtex.

Dans une variante, le fil de maille a un titrage compris entre 15 dtex et 500 dtex, de préférence entre 20 dtex et 300 dtex.

Le fil de maille peut être constitué d'un fil d'âme élastique, notamment un fil d'élasthanne, recouvert d'un ou de plusieurs fils de couverture, notamment par guipage, lesdits fils de couverture étant de préférence non élastiques.

Dans une variante, le fil de trame élastique a un titrage au moins trois fois supérieur à celui du fil de maille, de préférence au moins cinq fois supérieur à celui du fil de maille.

Cette disposition permet d'ajuster l'effet compressif exercé par ledit article.

Dans une variante, le bord côte a une hauteur (h) au minimum de 1 mm.

De préférence, la hauteur (h) du bord côte est supérieure ou égale à 20 mm, encore de préférence supérieure ou égale à 50 mm.

### Brève description des dessins

La présente invention sera mieux comprise à la lecture d'un exemple de réalisation cité à titre non limitatif, et illustrés par les figures suivantes dans lesquels :
- La figure **1** représente de façon schématique un article tubulaire de compression selon l'invention, en particulier une chaussette de compression ;
- La figure **2** représente de façon schématique l'armure de tricotage de la tige et du pied de la chaussette de compression représentée à la figure **1** **;**
- La figure **3** représente de façon schématique l'armure de tricotage du talon et de la pointe de pied de la chaussette de compression représentée à la figure **1** ;
- La figure **4** représente un graphique comparant les forces et allongements à rupture obtenus pour la tige et le bord côte de l'article décrit à la figure **1** avec les forces et allongements à rupture de la tige et du revers d'un article de compression de l'état de la technique, lesdits articles de compression étant classés comme appartenant à la classe de compression médicale II ;
- La figure **5A** représente des courbes comparant les forces à appliquer pour enfiler un article de compression selon l'invention et un article de compression de l'état de la technique ;
- La figure **5B** représente des courbes comparant les forces et allongements obtenus lorsque l'article de compression selon l'invention et un article de compression de l'état de la technique sont portés ;
- La figure **6** représente deux courbes comparant les allongements obtenus sous l'effet d'un poids déterminé pour un article de compression selon l'invention (courbe (**C**)), et un article de compression de l'état de la technique (courbe (**D**)) ;
- Les figures **7A** à **7D** représentent des histogrammes comparant les propriétés d'absorption de l'humidité (figure **7A**), d'évacuation de l'humidité (figure **7B**), de séchage (figure **7C**), et de rétention de l'humidité (figure **7D**) entre les chaussettes de compression selon l'invention et de l'état de la technique décrites ci-dessus en référence aux figures **1** à **6**.

### Description détaillée de l'invention

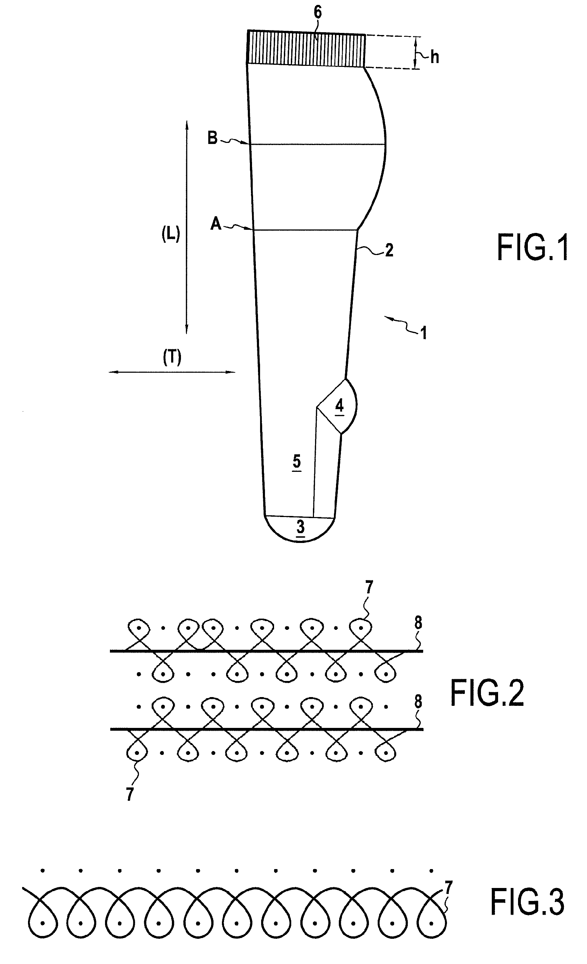

L'article tubulaire de compression **1** représenté à la figure **1** est une chaussette comprenant une tige **2**, une pointe de pied **3**, un talon **4**, un pied **5** et un bord côte **6** dans le prolongement de la tige **2**.

L'ensemble de l'article **1** est tricoté sur un métier à tricoter double cylindres, i.e. comprenant des cylindres supérieur et inférieur superposés travaillant chacun avec un nombre déterminé d'aiguilles (m).

L'article tubulaire **1** comprend une direction longitudinale (**L**) et une direction transversale (**T**) correspondant respectivement au sens des colonnes de mailles et au sens des rangées de mailles.

La tige **2**, le pied **5** et le bord côte **6** comprennent plusieurs rangées de mailles de côte respectivement (n)*(p), (n')* (p') et (n")*(p") tricotés avec un fil de maille **7** représenté à la figure **2**, entre lesquelles sont insérés des fils de trame élastiques **8** toutes les 1/1 à 1/5 rangées de mailles. Dans cet exemples précis, le fil de trame élastique **8** est inséré toutes les 1/1 à 1/2 rangées de mailles en côte (n)*(p) et (n')*(p'), de préférence entre toutes les rangées de mailles côte, dans la tige **2** et le pied **5** sans former de charge, ni de maille sur au moins 50 % en nombre du nombre d'aiguilles (m) travaillées, dans cet exemple précis sans former de charge, ni de maille sur toutes les aiguilles travaillées (m). La densité du fil de trame **8** est moins importante dans le bord côte **6** puisque le fil de trame **8** est inséré de préférence toutes les 1/2 ou plus rangées de mailles côte (n")*(p").

Le fil de trame élastique **8** peut être inséré entre deux rangées de mailles côtelées en formant quelques charges selon l'effet désiré, de préférence le fil de trame **8** ne forme aucune charge et est simplement posé entre les aiguilles du cylindre supérieure et les aiguilles du cylindre inférieur.

De préférence, le bord côte **6**, la tige **2** et le pied **5** ne comprennent que des mailles côtelées, en particulier la tige **2** et le pied **5** comprennent des rangées de côte 2/4 alternées avec des rangées de côte 1/1, les armures de tricotage sont ainsi représentées à la figure **2**.

De préférence, le talon **4** et la pointe de pied **3** ne comprennent pas un fil de trame élastique inséré en trame et sont tricotés à partir du fil de maille **8** selon des mailles jersey, de préférence des mailles jersey endroit dont l'armure de tricotage est représentée à la figure **3**. Les points noirs représentent, sur les figures **2** et **3**, les aiguilles des cylindres supérieur et inférieur.

La tige **2** comprend deux zones circulaires **A** et **B** consécutives, dont la hauteur est de l'ordre de 5 mm.

A titre d'exemple précis, la chaussette de compression **1** selon l'invention est tricotée dans son ensemble à partir du fil de maille **7** comprenant un fil d'âme élastique, en particulier un fil d'élasthanne de 44 dtex, recouvert d'un premier fil de couverture, notamment un fil en polyamide 6-6 à deux bouts de chacun 78 dtex, et d'un second fil de couverture, notamment en coton à un bout de 120 Nm ; et d'un fil de trame **8** (uniquement pour la tige, le pied et le bord côte), comprenant un fil d'âme élastique, en particulier un fil d'élasthanne de 330 dtex, recouvert d'un premier fil de couverture, notamment un fil en polyamide à un bout de 22 dtex, et d'un second fil de couverture, notamment en coton à un bout de 160 Nm.

Dans le but de tester les performances d'élasticité de la chaussette de compression selon l'invention, une chaussette de l'état de la technique est tricotée sur un métier à tricoter simple cylindre (i.e. cylindre-plateau de report, également désigné sous le terme « cylinder-dial ») en sorte de présenter sensiblement les mêmes dimensions, i.e. le même taillant, pour la même classe de compression médicale, à savoir la classe II ainsi qu'un revers formé d'une double épaisseur. La chaussette de compression de l'état de la technique est ainsi tricotée à partir d'un fil de maille comprenant un fil d'âme élastique, en particulier un fil d'élasthanne de 22 dtex, recouvert d'un premier fil de couverture, notamment à un bout de 78 dtex en polyamide 6-6 et d'un second fil de couverture, notamment en coton à un bout 160 Nm ; et d'un fil de trame comprenant un fil d'âme élastique, notamment un fil d'élasthanne de 330 dtex, recouvert d'un premier fil de couverture, notamment en polyamide 6-6 à un bout de 22 dtex et d'un second fil de couverture, notamment en coton à un bout de 160 Nm pour le revers, la tige et le pied. Pour la pointe de pied et le talon, la chaussette de l'état de la technique ne comprend pas de fil de trame élastique mais un fil de maille comprenant un fil d'âme élastique, en particulier un fil d'élasthanne de 78 dtex, recouvert d'un premier fil de couverture, notamment un fil en polyamide 6-6 à un bout de 78 dtex, et d'un second fil de couverture, notamment un fil en coton à un bout de 160 Nm. Pour réaliser une tige et un pied côtelés, le fil de maille est tricoté en jersey tandis que le fil de trame réalise des charges avec des rapports de 1/2, 1/3 et 2/3.

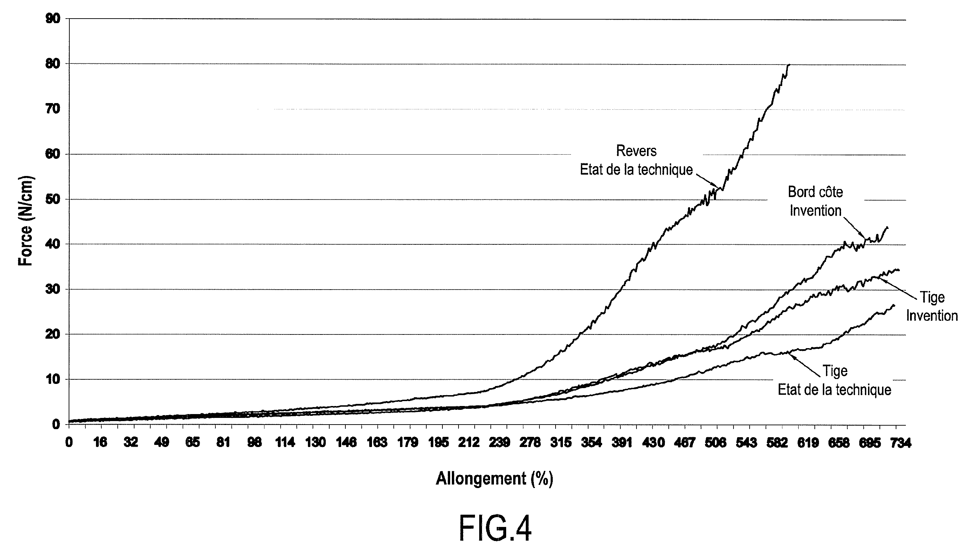

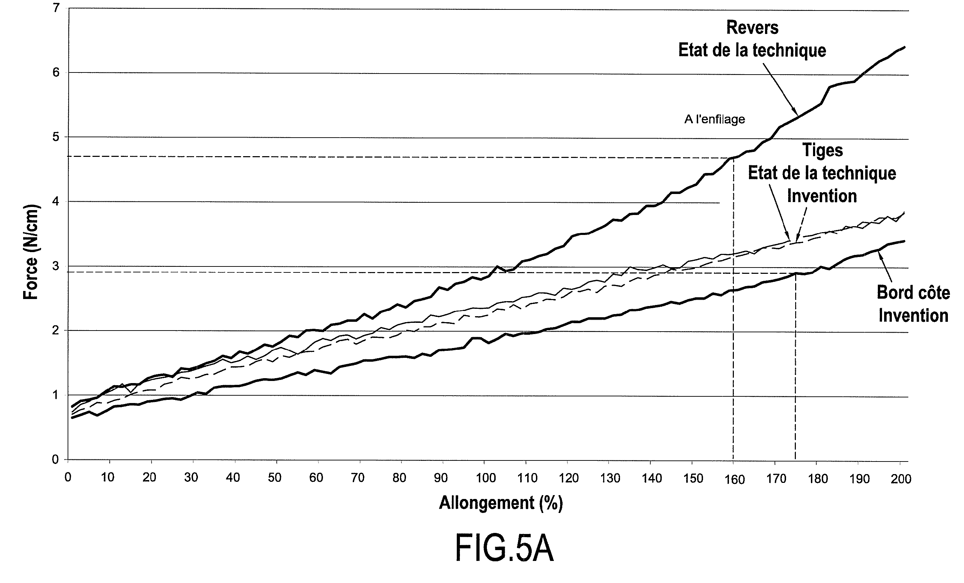

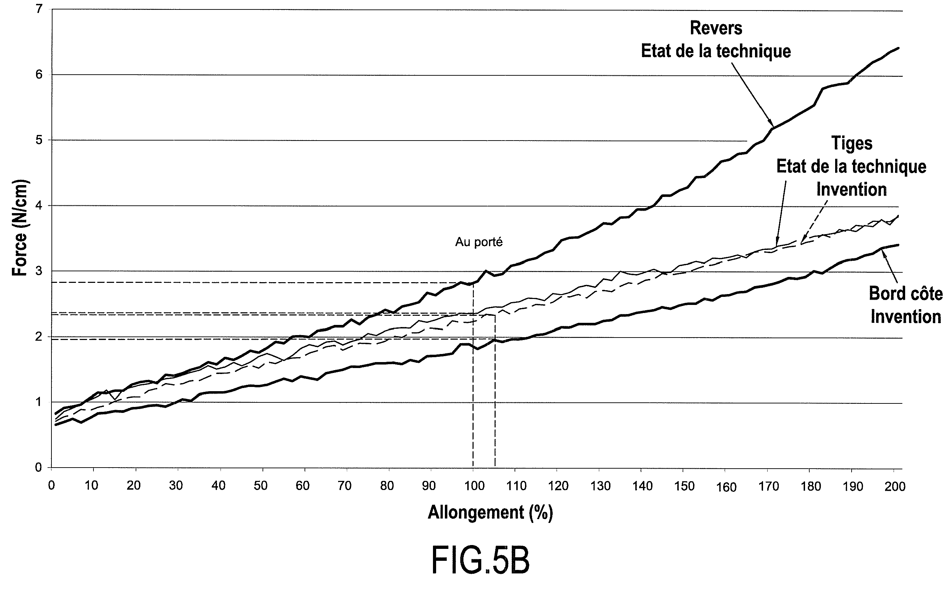

Les courbes de forces et allongements représentées aux figures **4**, **5A** et **5B** reprennent les valeurs de mesure effectuées selon la norme NF 14-704-1 de Juin 2005. La force de compression à la cheville exercée par la chaussette de compression selon l'invention et la chaussette de l'état de la technique sont équivalentes et mesurées selon la norme NF G30-102 B en date d'octobre 1986). La largeur à plat et au repos de la chaussette **1** selon l'invention mesurée au début du bord côte sur la tige est de 95 mm ; elle est de 90 mm concernant la largeur au début du revers sur la tige de la chaussette de compression de l'état de la technique. Les chaussettes de compression à tester sont disposées sur une jambe Hohenstein afin qu'elles soient en conformité avec leur placement au porté. Sur chacune des chaussettes, la hauteur du bord côte (**h1**) et la hauteur du revers (**h2**) sont mesurées et reportées sous ces derniers sur la tige. Des bandes annulaires de bord côte et de revers ainsi que de tige, respectivement de hauteur (**h1**) et (**h2**) sont découpées dans chaque chaussette de compression. Ces bandes annulaires sont ouvertes en sorte de former des éprouvettes de test rectangulaires dont les largeurs correspondent aux hauteurs (**h1**) et (**h2**) au porté. Chacune de ces éprouvettes sont placées sur un dynamomètre et évaluées selon la norme NF 14-704-1 précitée. L'écartement entre les mors du dynamomètre a été ajusté à 50 mm.

La mesure des forces et allongements est réalisée en conformité avec le comportement au porté des chaussettes de compression ; ainsi le revers est testé en double épaisseur tandis que le bord côte et les bandes de tige sont testés en simple épaisseur.

On remarque ainsi que le bord côte **6** présente un allongement à rupture (716 % environ) supérieur à celui du revers de l'état de la technique (596 % environ). Par ailleurs, les comportements élastiques de la tige de la chaussette de l'état de la technique et de son revers divergent ; on retrouve ainsi l'effet garrot du revers critiqué. A l'opposé, les comportements élastiques du bord côte et de la tige de la chaussette selon l'invention sont très proches (respectivement 716 % et 728 % d'allongements à rupture) permettant ainsi d'obtenir une dégressivité de la compression exercée très homogène.

On remarque également que la force à rupture de la tige de la chaussette de compression selon l'invention (34,5 N/cm) est supérieure de 29 % à la force à rupture de la tige de la chaussette de l'état de la technique (26,7 N/Cm). Une explication non exhaustive de cet effet est que les fils de trame élastiques ne forment pas de charge, ni de maille, dans la chaussette de compression selon l'invention, ou éventuellement très peu, tandis que dans la chaussette de l'état de la technique, les fils de trame forment des charges à intervalles très réguliers, lesdites charges formant des points d'ancrage et donc de blocage de la structure maillée.

Le tableau **1** ci-dessous reprend des valeurs extraites des figures **5A** et **5B** représentant le comportement des chaussettes de compression selon l'invention et de l'état de la technique selon les plages d'utilisation, i.e. au porté sous un allongement allant de 100 % à 105 % et à l'enfilage sous un allongement allant de 160 % à 175 %.

On remarque ainsi que deux allongements différents sont reportés sur les figures **5A** et **5B** puisque les largeurs des zones testées au repos sont différentes pour l'article de compression selon l'invention (90 mm) et l'article de compression de l'état de la technique (95 mm). Ces différences de longueurs sont dues aux métiers à tricoter utilisés : simple cylindre ou double cylindres.

**Tableau 1**

| | | **Chaussette de l'état de la technique** | **Chaussette selon l'invention** |
|---|---|---|---|
| **Au repos** | Largeur borde côte ou revers (mm) | **95** | **90** |
| | Circonférence (mm) | **190** | **180** |
| **Au porté** | Allongement (%) | **100** | **105** |
| | Force (N/cm) bord côte ou revers | **2.88** | **2.03** |
| | Force (N/cm) tige | **2.34** | **2.35** |
| **A l'enfilage** | Allongement (%) | **160** | **175** |
| | Force (N/cm) bord côte ou revers | **4.72** | **2.86** |
| | Force (N/cm) tige | **3.18** | **3.38** |

La force nécessaire (**N/cm**) pour enfiler le bord côte est inférieure de 65 % à celle nécessaire pour enfiler le revers de la chaussette de compression de l'état de la technique. Lorsque la chaussette de compression selon l'invention est portée, le bord côte exerce une force (**N/cm**) inférieure de 16 % à celle exercée par la tige, la dégressivité de la compression exercée est ainsi parfaitement respectée. Au contraire, pour la chaussette de compression de l'état de la technique, le revers exerce une force (**N/cm**) supérieure de 23 % à celle exercée par la tige, la dégressivité de la compression exercée n'est ainsi pas parfaitement assurée. Bien sûr, du fait de la morphologie de la jambe, le rayon de courbure étant plus important au niveau de la portion de la tige recouvrant la jambe que celui de la portion de la tige recouvrant la cheville, on obtient tout de même entre la cheville et la jambe une dégressivité globale de la compression exercée. Cependant, cette dégressivité n'est pas parfaitement régulière et donc homogène entre la cheville et la jambe.

La chaussette de compression selon l'invention permet d'améliorer le confort en évitant que le fil de trame ne forme des flottés importants sur la face envers de ladite chaussette, cette dernière disposition combinée avec un bord côte facilite également l'enfilage de ladite chaussette **1**.

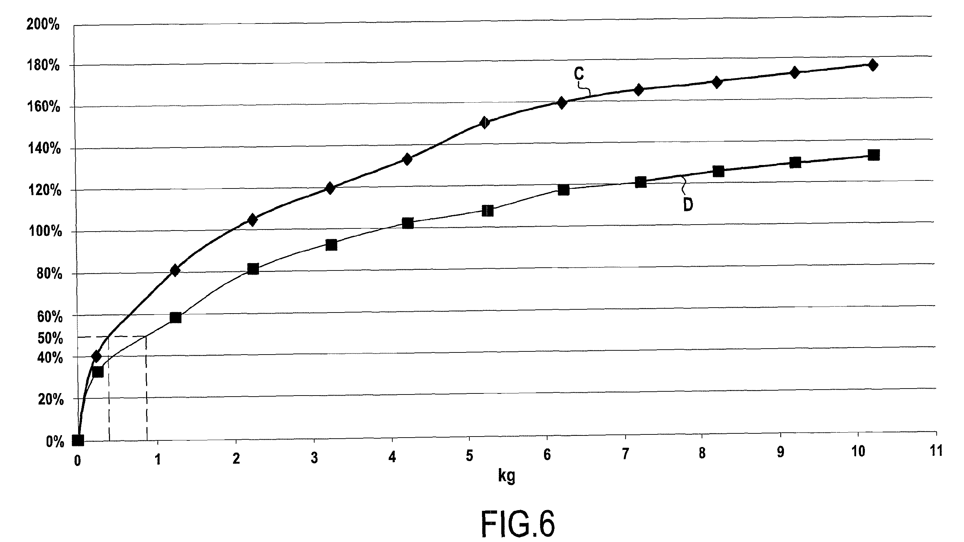

La figure **6** représente deux courbes (**C,D**) obtenues par la méthode de test d'extensibilité décrite ci-après effectuée sur les tiges de la chaussette de compression **1** selon l'invention et la chaussette de compression de l'état de la technique testée en référence aux figures **4**, **5A** et **5B**, toutes deux ayant un niveau de compression de classe II.

Les deux chaussettes testées ont la même hauteur de tige entre le talon et le début du revers ou du bord côte, soit 21 cm. Les deux chaussettes ont également le même taillant, la circonférence au niveau de la cheville est de 27,5 cm et la circonférence au niveau du mollet est de 41,5 cm. La hauteur des chaussettes est de 44 cm.

Les chaussettes sont suspendues sur un banc d'extension au moyen d'une première pince pinçant les chaussettes sur la tige au début du revers ou du bord côte. Une seconde pince, mobile par rapport au banc d'extension, est solidarisée aux chaussettes dans la partie inférieure des tiges au niveau de la démarcation du talon. Des poids sont accrochés sur la seconde pince de manière à provoquer une extension dans la direction verticale des tiges desdites chaussettes. Le banc d'extension est muni d'une graduation permettant de mesurer l'allongement obtenu selon le poids total appliqué au niveau de la seconde pince.

La masse de la seconde pince est de 0,250 Kg et la masse de chaque poids est de 1 Kg. La mesure de l'extension des tiges est relevée pour la seconde pince seule, puis en ajoutant au fur et à mesure un poids de 1 Kg jusqu'à 10 Kg au total.

On considère que lorsque les chaussettes de compression sont portées par l'utilisateur, les tiges sont étirées d'au moins 50 % de leurs longueurs au repos.

On observe ainsi sur la figure **6** que pour un allongement de 50 %, la force (**Kg**) à exercer pour amener la tige à sa dimension d'application est inférieure pour la chaussette de compression selon l'invention que celle nécessaire pour la chaussette de compression de l'état de la technique. Or, cette force (**Kg**) est également celle qui est appliquée au bord côte ou au revers lorsque la chaussette de compression est placée sur la jambe. La force ou traction exercée sur la chaussette de compression selon l'invention dans le sens transverse (**T**) est environ égale à 50 % de la force exercée sur la chaussette de compression de l'état de la technique pour un même allongement (**%**).

On peut ainsi en conclure que la chaussette de compression obtenue par le procédé selon l'invention a une meilleure tenue sur la jambe que la chaussette de compression de l'état de la technique. En effet, la chaussette de compression selon l'invention aura beaucoup moins tendance à glisser sur la jambe que la chaussette de compression de l'état de la technique. Cet effet technique s'explique par la construction de la chaussette de compression selon l'invention, laquelle comprend un fil de trame disposé librement entre deux rangées de mailles consécutives, au moins sur 50 % en nombre du nombre (m) des aiguilles tricotées sans former de charge, ni de maille. Le maintien d'une chaussette de compression en place au porté est un équilibre entre la compression exercée transversalement et l'allongement exercé par la jambe sur la chaussette entre le bord côte ou revers de la chaussette et la cheville.

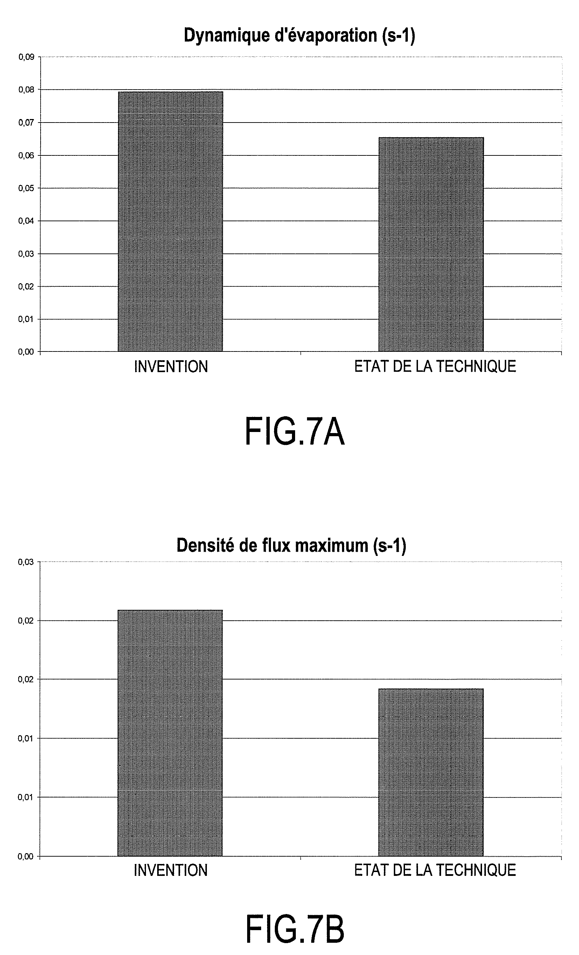

Les histogrammes représentés aux figures **7A** à **7D** reprennent des mesures effectuées sur les chaussettes de compression selon l'invention et de l'état de la technique décrites ci-dessus et de classe II selon la méthode de mesure décrite ci-après. Les mesures sont effectuées sur le bord côte de la chaussette selon l'invention (simple épaisseur) et sur le revers (double épaisseur) de la chaussette de l'état de la technique.

La composition en poids desdites chaussettes est sensiblement équivalente :
- 47 % de coton, 43 % de polyamide et 10 % d'élasthanne pour la chaussette selon l'invention,
- 50 % de coton, 38 % de polyamide, 12 % d'élasthanne pour la chaussette de l'état de la technique.

Les chaussettes de compression ont été lavées de manière à éliminer tous les résidus issus des procédés de fabrication. Les échantillons de chaussette ont été prélevés sur une jambe normalisée du type Hohenstein.

Les échantillons, lors des mesures, sont étirés d'au moins 50 % de leurs longueurs au repos, ce qui correspond au comportement des chaussettes de compression au porté.

Cette méthode de test a ainsi pour but d'étudier la dynamique du transfert de vapeur d'eau à travers une pièce textile qui a été préalablement mise en contact avec une quantité d'eau connue, en particulier 1 mg d'eau, ce qui correspond à une goutte de sueur. La densité de flux de vapeur d'eau dégagée par l'échantillon testé est mesurée et étudiée en fonction du temps. Le dispositif sur lequel le test est effectué comprend un support chauffant chauffé à une température correspondant à la température corporelle (35°C), un porte échantillon, et une cellule de mesure, telle qu'une cellule de mesure Peltier équipée d'un régulateur. Le support, monté en vis-à-vis du porte échantillon, est de préférence en cuivre et peut être recouvert d'une couche de latex en sorte de reproduire la peau humaine. Le porte échantillon est réalisé de façon à assurer l'étanchéité de l'ensemble formé du support, de l'échantillon et de la cellule de mesure. Ainsi, les variations d'humidité et de température ambiantes n'ont pas d'incidence sur la mesure. La cellule de mesure comprend un capteur de flux et un condenseur permettant l'évacuation de l'humidité.

L'évolution temporelle de la densité de flux de vapeur d'eau à travers les échantillons comprend les étapes suivantes : mise en contact de l'échantillon avec une goutte d'eau correspondant à la phase d'absorption, phase de transfert de l'humidité dans l'échantillon ou de diffusion puis phases d'évaporation et de séchage.

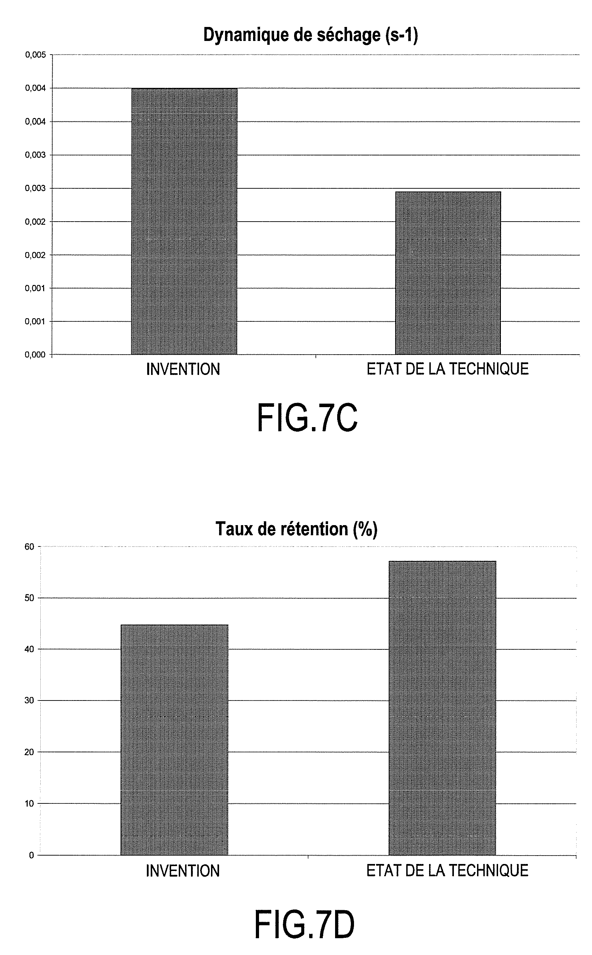

La figure **7A** correspond à la phase d'absorption de l'humidité lors de la mise en contact des échantillons avec une goutte d'eau. Les figures **7B** et **7C** correspondent respectivement à la capacité des échantillons à évacuer l'humidité basée sur la densité de flux de vapeur d'eau maximale et la dynamique de séchage. Enfin, la figure **7D** correspond au taux de rétention de la quantité d'eau maintenue dans les échantillons après l'essai, c'est-à-dire après leur séchage.

On remarque à la lecture de ces histogrammes que la chaussette de compression selon l'invention est supérieure en tous points à la chaussette de compression de l'état de la technique. Le bord côte de la chaussette de compression selon l'invention absorbe ainsi plus rapidement l'humidité, évacue et sèche plus rapidement que le revers de la chaussette de compression de l'état de la technique. En outre, le bord côte de la chaussette de compression selon l'invention retient moins au final l'humidité que le revers de la chaussette de l'état de la technique une fois le test terminé.

En conclusion, la chaussette selon l'invention améliore de façon importante le confort thermique de l'utilisateur.

## Revendications

1. Procédé de fabrication d'un article tubulaire de compression (1), du type chaussette (1), bas ou mi-bas, ou collant, ayant au moins une tige (2), une pointe de pied (3), un talon (4), un pied (5) et un bord côte (6) dans le prolongement de la tige (2) délimitant une ouverture pour l'introduction du pied dans ledit article (1), comprenant les étapes suivantes :
a. Une première étape de tricotage de la tige (2), du pied (5) et du bord côte (6) avec au moins un fil de maille (7) sur un métier à tricoter double cylindres comprenant un cylindre supérieur et un cylindre inférieur travaillant chacun avec (m) aiguilles, au cours de laquelle plusieurs rangées de mailles en côte (n)*(p), (n')*(p') et (n")*(p") sont tricotées respectivement pour la tige (2), le pied (5) et le bord côte (6), (m), (n), (n'), (n"), (p), (p') et (p") étant des nombres entiers supérieurs ou égaux à 1,
b. Une étape d'insertion au cours de la première étape de tricotage d'un fil de trame élastique (8) entre deux rangées de mailles côtelées (n)*(p) et (n')*(p') toutes les 1/1 à 1/5 rangées de mailles de la tige (2) et du pied (5) et toutes les 1/2 à 1/5 rangées de mailles du bord côte (6), sur au moins 50 % en nombre du nombre (m) d'aiguilles sans former de charge, ni de maille, la densité en fil de trame (8) dans le bord côte (6) étant inférieure ou égale à la densité en fil de trame dans la tige (2),
c. Une seconde étape de tricotage de la pointe de pied (3) et du talon (4) sur ledit métier à tricoter double cylindres avec au moins un fil de maille (7) et éventuellement un fil de trame élastique.

2. Procédé de fabrication selon la revendication 1, **caractérisé en ce que** la seconde étape de tricotage ne comprend pas l'insertion en trame d'un fil de trame élastique.

3. Procédé de fabrication selon l'une ou l'autre des revendications 1 et 2, **caractérisé en ce qu'**il comprend au cours de la dite première étape et/ou de ladite seconde étape, une sous-étape de tricotage du fil de maille (7) pour la formation d'une ou plusieurs rangées de mailles en jersey avant et/ou arrière.

4. Procédé de fabrication selon l'une quelconque des revendications 1 à 3, **caractérisé en ce que** le bord côte (6) a une hauteur (h) au minimum de 1 mm.

5. Procédé de fabrication selon l'une quelconque des revendications 1 à 4, **caractérisé en ce que** la tige (2) présente deux zones circulaires distinctes A et B consécutives, et **en ce qu'**au cours de la première étape de tricotage, la tension exercée sur le fil de trame élastique (8) lors du tricotage de la zone A est supérieure à la tension exercée sur le fil de trame élastique (8) dans la zone B, en sorte que la circonférence de la zone circulaire A soit inférieure à la circonférence de la zone circulaire B.

6. Procédé de fabrication selon l'une quelconque des revendications 1 et 3 à 5, **caractérisé en ce qu'**il comprend au cours de la première étape et/ou de la seconde étape, une sous-étape au-cours de laquelle le fil de trame élastique (8) effectue une ou plusieurs charges.

7. Procédé de fabrication selon l'une quelconque des revendications 1 à 6, **caractérisé en ce que** le fil de trame élastique (8) est inséré au cours de la première étape toutes les 1/1 à 1/2 rangées de mailles.

8. Article tubulaire à effet compressif (1), du type chaussette, bas ou mi-bas, ou collant, ayant au moins une tige (2), une pointe de pied (5), un talon (4), un pied (5), et un bord côte (6) dans le prolongement de la tige (2) délimitant une ouverture pour l'introduction du pied (5) dans ledit article (1), obtenu par la mise en oeuvre du procédé selon l'une quelconque des revendications 1 à 7, comprenant un fil de maille (7) tricoté en côte (n)*(p), (n')*(p') et (n") *(p") respectivement dans la tige (2), le pied (5) et le bord côte (6), (n), (n'), (n"), (p), (p') et (p") étant des nombres entiers supérieurs ou égaux à 1, et un fil de trame élastique (8) disposé entre deux rangées de mailles consécutives toutes les 1/1 à 1/5 rangées de mailles dans la tige (2), le pied (5) et le bord côte (6), au moins sur 50 % en nombre du nombre de colonnes de mailles sans former de charge, ni de maille, la densité en fil de trame (8) dans le bord côte (6) étant inférieure ou égale à la densité en fil de trame dans la tige (2),

9. Article tubulaire (1) selon la revendication 8, **caractérisé en ce que** le fil de trame élastique (8) est inséré en trame dans la tige (2) et le pied (5) toutes les 1/1 à 1/2 rangées de mailles.

10. Article tubulaire (1) selon l'une ou l'autre des revendications 8 et 9, **caractérisé en ce que** le fil de trame (8) est constitué d'un fil d'élasthanne recouvert d'au moins deux fils.

11. Article tubulaire (1) selon l'une quelconque des revendications 8 à 10, **caractérisé en ce que** le fil de trame élastique (8) a un titrage compris entre 150 dtex et 2000 dtex, de préférence entre 300 dtex et 1500 dtex.

12. Article tubulaire (1) selon l'une quelconque des revendications 8 à 11, **caractérisé en ce que** le fil de maille (7) a un titrage compris entre 15 dtex et 500 dtex, de préférence entre 20 dtex et 300 dtex.

13. Article tubulaire (1) selon l'une quelconque des revendications 8 à 12, **caractérisé en ce que** le fil de trame élastique (8) a un titrage au moins trois fois supérieur à celui du fil de maille (7), de préférence au moins cinq fois supérieur à celui du fil de maille (7).

14. Article tubulaire selon l'une quelconque des revendications 8 à 13, **caractérisé en ce que** le bord côte a une hauteur (h) au minimum de 1 mm.

## Patentansprüche

1. Verfahren zur Herstellung eines röhrenförmigen Kompressionsartikels (1) vom Typ flache oder halbhohe Socke (1) oder Strumpf mit mindestens einem Schaft (2), einer Fußspitze (3), einer Ferse (4) einem Fuß (5) und einem Bund (6) in der Verlängerung des Schaftes (2), der eine Öffnung für die Einführung des Fußes in den Artikel (1) begrenzt, umfassend die folgenden Schritte:
a. einen ersten Schritt des Strickens des Schaftes (2), des Fußes (5) und des Bundes (6) mit mindestens einem Strickgarn (7) auf einer Doppelzylinder-Strickmaschine, umfassend einen oberen Zylinder und einen unteren Zylinder, die jeweils mit (m) Nadeln arbeiten, während dessen mehrere Reihen von Rippenstrickmaschen (n)*(p), (n')*(p') und (n")*(p") für den Schaft (2), den Fuß (5) und den Bund (6) gestrickt werden, wobei (m), (n), (n'), (n"), (p), (p'), (p") ganze Zahlen größer oder gleich 1 sind,
b. einen Schritt des Einsetzens während des ersten Schrittes des Strickens eines elastischen Schussfadens (8) zwischen zwei Reihen von Rippenstrickmaschen (n)*(p) und (n')*(p') alle 1/1 bis 1/5 Maschenreihen des Schafts (2) und des Fußes (5) und alle 1/2 bis 1/5 Maschenreihen des Bundes (6) auf zahlenmäßig mindestens 50% der Nadelzahl (m) ohne Bildung einer Charge oder Masche, wobei die Schussfadendichte (8) im Bund (6) kleiner oder gleich der Schussfadendichte im Schaft (2) ist,
c. einen zweiten Schritt des Strickens der Fußspitze (3) und der Ferse (4) auf der Doppelzylinder-Strickmaschine mit mindestens einem Strickgarn (7) und eventuell einem elastischen Schussfaden.

2. Herstellungsverfahren nach Anspruch 1, **dadurch gekennzeichnet, dass** der zweite Strickschritt kleinen Schusseintrag eines elastischen Schussfadens umfasst.

3. Herstellungsverfahren nach dem einen oder dem anderen der Ansprüche 1 und 2, **dadurch gekennzeichnet, dass** es während des ersten Schrittes und/oder des zweiten Schrittes einen Unterschritt des Strickens des Wirkgarns (7) zur Bildung einer oder mehrerer Reihen von Jerseymaschen vorne und/oder hinten umfasst.

4. Herstellungsverfahren nach einem der Ansprüche 1 bis 3, **dadurch gekennzeichnet, dass** der Bund (6) eine Höhe (h) von mindestens 1 mm hat.

5. Herstellungsverfahren nach einem der Ansprüche 1 bis 4, **dadurch gekennzeichnet, dass** der Schaft (2) zwei getrennte aufeinanderfolgende kreisförmige Zonen A und B aufweist, und dass während des ersten Strickschrittes die auf den elastischen Schussfaden (8) beim Tricken der Zone A ausgeübte Spannung größer als die auf den elastischen Schussfaden (8) in der Zone B ausgeübte Spannung ist, so dass der Umfang der kreisförmigen Zone A kleiner als der Umfang der kreisförmigen Zone B ist.

6. Herstellungsverfahren nach einem der Ansprüche 1 und 3 bis 5, **dadurch gekennzeichnet, dass** es während des ersten und/oder des zweiten Schrittes einen Unterschritt umfasst, während dessen der elastische Schussfaden (8) eine oder mehrere Chargen ausführt.

7. Herstellungsverfahren nach einem der Ansprüche 1 bis 6, **dadurch gekennzeichnet, dass** der elastische Schussfaden (8) während des ersten Schrittes alle 1/1 bis 1/2 Maschenreihen eingesetzt wird.

8. Röhrenförmiger Artikel mit Kompressionswirkung (1) vom Typ flache oder halbhohe Socke oder Strumpf mit mindestens einem Schaft (2), einer Fußspitze (5), einer Ferse (4) einem Fuß (5) und einem Bund (6) in der Verlängerung des Schaftes (2), der eine Öffnung für die Einführung des Fußes (5) in den Artikel (1) begrenzt, hergestellt durch den Einsatz des Verfahrens nach einem der Ansprüche 1 bis 7, umfassend ein Strickgarn (7), das im Rippenstrick (n)*(p), (n')*(p') und (n")*(p") im Schaft (2), Fuß (5) und Bund (6) gestrickt ist, wobei (m), (n), (n'), (n"), (p), (p'), (p") ganze Zahlen größer oder gleich 1 sind, und einen elastischen Schussfaden (8), der zwischen zwei aufeinanderfolgenden Maschenreihen alle 1/1 bis 1/5 Maschenreihen im Schaft (2), Fuß (5) und Bund (6) auf zahlenmäßig mindestens 50 % der Maschenstäbchen, ohne eine Charge oder Masche zu bilden, angeordnet ist, wobei die Schussfadendichte (8) im Bund (6) kleiner oder gleich der Schussfadendichte im Schaft (2) ist.

9. Röhrenförmiger Artikel (1) nach Anspruch 8, **dadurch gekennzeichnet, dass** ein Schusseintrag des elastischen Schussfadens (8) im Schaft (2) und Fuß (5) alle 1/1 bis 1/2 Maschenreihen erfolgt.

10. Röhrenförmiger Artikel (1) nach dem einen oder dem anderen der Ansprüche 8 und 9, **dadurch gekennzeichnet, dass** der Schussfaden (8) von einem mit mindestens zwei Garnen bedeckten Elasthan-Faden gebildet ist.

11. Röhrenförmiger Artikel (1) nach einem der Ansprüche 8 bis 10, **dadurch gekennzeichnet, dass** der elastische Schussfaden (8) einen Titer zwischen 150 dtex und 2000 dtex, vorzugsweise zwischen 300 dtex und 1500 dtex, hat.

12. Röhrenförmiger Artikel (1) nach einem der Ansprüche 8 bis 11, **dadurch gekennzeichnet, dass** das Strickgarn (7) einen Titer zwischen 15 dtex und 500 dtex, vorzugsweise zwischen 20 dtex und 300 dtex, hat.

13. Röhrenförmiger Artikel (1) nach einem der Ansprüche 8 bis 12, **dadurch gekennzeichnet, dass** der elastische Schussfaden (8) einen mindestens dreimal größeren Titer als jener des Strickgarns (7), vorzugsweise mindestens fünfmal größer als jener des Strickgarns (7), hat.

14. Röhrenförmiger Artikel nach einem der Ansprüche 8 bis 13, **dadurch gekennzeichnet, dass** der Bund eine Höhe (h) von mindestens 1 mm hat.

## Claims

1. A method for producing a tubular compression item (1), of sock (1), stocking or knee-length stocking, or panty hose type having at least one leg part (2), one foot tip (3), one heel (4), one foot (5) and one ribbed edge (6) in the extension of the leg part (2) delimiting an opening for introducing the foot into said item (1), comprising the following steps:
a. a first step for knitting the leg part (2), the foot (5) and the ribbed edge (6) with at least one knitting yarn (7) on a double cylinder knitting machine comprising an upper cylinder and a lower cylinder each operating with (m) needles, during which several rows of ribbed stitches (n)*(p), (n')*(p') and (n")*(p") are respectively knitted for the leg part (2), the foot (5) and the ribbed edge (6), (m), (n), (n'), (n"), (p), (p') and (p") being integers greater than or equal to 1,
b. a step for inserting during the first knitting step an elastic weft yarn (8) between two rows of ribbed stitches (n)*(p) and (n')*(p') every 1/1 to 1/5 rows of stitches of the leg part (2) and of the foot (5) and every 1/2 to 1/5 rows of stitches of the ribbed edge (6), on at least 50% by number of the number (m) of needles without forming any tuck stitch, or loop stitch, the weft yarn density (8) in the ribbed edge (6) being less than or equal to the weft yarn density in the leg part (2),
c. a second step for knitting the foot tip (3) and the heel (4) on said double cylinder knitting machine with at least one knitting yarn (7) and optionally an elastic weft yarn.

2. The production method according to claim 1, **characterized in that** the second knitting step does not comprise the weft insertion of an elastic weft yarn.

3. The production method according to either one of claims 1 and 2, **characterized in that** it comprises during said first step and/or during said second step, a sub-step for knitting the knitting yarn (7) in order to form one or several rows of plain and/or purl jersey stitches.

4. The production method according to any one of claims 1 to 3, **characterized in that** the ribbed edge (6) has a height (h) at least of 1 mm.

5. The production method according to any one of claims 1 to 4, **characterized in that** the leg part (2) has two consecutive distinct circular areas A and B, and **in that** during the first knitting step, the tension exerted on the elastic weft yarn (8) during the knitting of the area A is greater than the tension exerted on the elastic weft yarn (8) in the area B, so that the circumference of the circular area A is less than the circumference of the circular area B.

6. The production method according to any one of claims 1 and 3 to 5, **characterized in that** it comprises during the first step and/or during the second step, a sub-step during which the elastic weft yarn (8) produces one or several tuck stitches.

7. The production method according to any one of claims 1 to 6, **characterized in that** the elastic weft yarn (8) is inserted during the first step every 1/1 to 1/2 rows of stitches.

8. A tubular item with a compressive effect (1), of sock, stocking or knee-length stocking or pantyhose type, having at least one leg part (2), one foot tip (5), one heel (4), one foot (5), and a ribbed edge (6) in the extension of the leg part (2) delimiting an opening for introducing the foot (5) into said item (1), obtained by applying the method according to any one of claims 1 to 7, comprising a knitting yarn (7) respectively (n)*(p), (n)*(p') and (n")*(p") in the leg part (2), the foot (5) and the ribbed edge (6), (n), (n'), (n"), (p), (p') and (p") being integers greater than or equal to 1, and an elastic weft yarn (8) positioned between two rows of consecutive stitches every 1/1 to 1/5 rows of stitches in the leg part (2), the foot (5) and the ribbed edge (6), at least on 50% by number of the number of columns of stitches without forming any tuck stitch, or loop stitch, the weft yarn density (8) in the ribbed edge (6) being less than or equal to the weft yarn density in the leg part (2).

9. The tubular item (1) according to claim 8, **characterized in that** the elastic weft yarn (8) is inserted as a weft in the leg part (2) and the foot (5) every 1/1 to 1/2 rows of stitches.

10. The tubular item (1) according to either one of claims 8 and 9, **characterized in that** the weft yarn (8) consists of an elastane yarn covered with at least two yarns.

11. The tubular item (1) according to any one of claims 8 to 10, **characterized in that** the elastic weft yarn (8) has a count comprised between 150 dtex and 2,000 dtex, preferably between 300 dtex and 1,500 dtex.

12. The tubular item (1) according to any one of claims 8 to 11, **characterized in that** the knitting yarn (7) has a count comprised between 15 dtex and 500 dtex, preferably between 20 dtex and 300 dtex.

13. The tubular item (1) according to any one of claims 8 to 12, **characterized in that** the elastic weft yarn (8) has a count at least three times greater than that of the knitting yarn (7), preferably at least five times greater than that of the knitting yarn (7).

14. The tubular item according to any one of claims 8 to 13, **characterized in that** the ribbed edge has a height (h) at least of 1 mm.
